# EUROPEAN PATENT APPLICATION

(11) **EP 3 603 602 A1**
(43) Date of publication of application: **05.02.2020**
(21) Application number: 18774868.6
(22) Date of filing: 28.03.2018
(51) Int. Cl.: A61J 1/00, A61M 5/28

(54) **PRE-FILLED SYRINGE PACKAGING BODY**

(30) Priority: 29.03.2017 JP 2017065820
(71) Applicant: Terumo Kabushiki Kaisha, Tokyo 151-0072 (JP)
(72) Inventor: MARUYAMA, Sayaka, Ashigarakami-gun Kanagawa 259-0151 (JP); ABE, Yoshihiko, Ashigarakami-gun Kanagawa 259-0151 (JP)
(74) Representative: Casalonga
(86) International application number: PCT/JP2018/012884
(87) International publication number: WO 2018/181531

(57) **Abstract**

Provided are a prefilled syringe package in which a prefilled syringe and a buffering material for buffering an external force applied to the prefilled syringe package by being deformed by the external force are disposed in a packaging material, a prefilled syringe package in which a prefilled syringe is accommodated in a packaging material having an action for buffering an external force applied to the prefilled syringe package by being deformed by the external force, or a prefilled syringe package in which the two are combined.

## Description

### Technical Field

The present invention relates to a package of a prefilled syringe which is prefilled with a liquid such as a drug solution or water for injection. More specifically, the present invention relates to a prefilled syringe package in which damage to the prefilled syringe or liquid leakage does not occur in a case where a large external force such as vibration during dropping from a high position or transporting is applied to the prefilled syringe package, and which can prevent or reduce aggregation of components in a drug solution with which a syringe is filled, falling off of silicone oil applied to an inner wall of a syringe main body from the syringe inner wall surface, aggregation of components contained in a drug solution in a syringe due to silicone oil fallen off from a wall surface, and the like in a case where low vibration, such as tapping during movement within a medical institution, placement of a prefilled syringe package that has been carried on a stand such as a table, or nursing performed in the vicinity of a place where a prefilled syringe package is placed, or other weak external forces are applied to the prefilled syringe package.

### Background Art

In recent years, a prefilled syringe in which a liquid such as a drug solution is accommodated in advance has been widely used since it has excellent convenience or rapidity because a predetermined drug solution can be directly administered to a patient as it is, and it has excellent safety, hygienic properties because there is no concern of contamination accompanied by preparation of a drug solution.

A so-called "blister packaging" in which a prefilled syringe is stored in a plastic outer container having a concave portion for storing the prefilled syringe and the plastic outer container is sealed has been widely used as a form of packaging a prefilled syringe (for example, PTLs 1 to 4).

The plastic outer container used for blister packaging is manufactured through vacuum molding, vacuum pressure molding, or compression molding using a single-layer or multiple-layer sheet or film made of one or more kinds of olefin resins such as polypropylene, polyethylene, and a cyclic olefin polymer, polyesters such as polyethylene terephthalate or polyethylene naphthalate, and thermoplastic resins such as polyamide, polystyrene, and polyvinyl chloride, or through injection molding using the thermoplastic resins.

A blister-packaged prefilled syringe is usually stored in a state where the syringe is fixed to a storage concave portion of a plastic outer container forming a blister packaging material so as not to move within the packaging material, and is designed so as to prevent damage caused even if the syringe vibrates during transportation or is dropped.

However, in the prefilled syringe package, the dropping from a high position, the vibration during transportation, or the like is not an external force that is frequently applied. Vibration or other external forces which are applied on a daily basis are slight jumping of the prefilled syringe package accompanied by dropping from a low position during movement of the prefilled syringe package within a medical institution, placement of the prefilled syringe package on a stand such as a table, or tapping (an action of rhythmically beating the back or the chest for making phlegm or a foreign substance easily come out of the body) performed in the vicinity of a place where the prefilled syringe package is placed.

The present inventors have studied how the application of low vibration or other small external forces, such as the slight jumping of the prefilled syringe package accompanied by the movement of the prefilled syringe package within a medical institution, the dropping from a low position during placement on a stand, and the tapping during nursing performed in the vicinity of a place where the prefilled syringe package is placed, which are applied to the prefilled syringe package on a daily basis and have not been considered problematic in the related art influences on the prefilled syringe package.

As a result, it has been found that the low vibration or other small external forces applied to the prefilled syringe package on a daily basis are less likely to cause a large accident such as damage to a prefilled syringe, but has become one of the factors causing a problem such as aggregation of components in a drug solution accommodated in a syringe, falling off of a liquid lubricant such as silicone oil applied to an inner wall surface of a syringe main body from the wall surface, and aggregation of components contained in a drug solution with which a syringe is filled due to silicone oil fallen off from the wall surface.

The problems such as aggregation of components in a drug solution accommodated in a syringe, falling off of silicone oil applied to an inner wall of a syringe main body from the wall surface, and aggregation of components contained in a drug solution in a syringe due to silicone oil fallen off from the wall surface cause harmful events such as a decrease in drug efficacy or side effects.

From the point, it is necessary to develop a prefilled syringe package that does not cause problems, such as aggregation of components contained in a drug solution accommodated in a syringe, falling off of silicone oil applied to an inner wall of a syringe main body from the wall surface, and aggregation of components contained in a drug solution in a syringe due to silicone oil fallen off from the wall surface, caused by not only large external forces such as vibration during dropping from a high position or transporting, but also low vibration or other small external forces which are often applied to the prefilled syringe package on a daily basis.

### Citation List

### Patent Literature

PTL 1: JP-A-2007-301033
PTL 2: JP-A-2008-104645
PTL 3: JP-A-2011-005182
PTL 4: JP-A-2011-006154
PTL 5: JP-A-2007-502310

### Summary of Invention

### Technical Problem

An object of the present invention is to provide a prefilled syringe package in which damage or liquid leakage due to a large external force, such as vibration during dropping from a high position or transporting, applied to the prefilled syringe package does not occur, and which can prevent or reduce occurrence of problems such as aggregation of components in a drug solution with which a syringe is filled, falling off of silicone oil applied to an inner wall surface of a syringe main body from the wall surface, and aggregation of components contained in a drug solution in a syringe due to the silicone oil fallen off from a wall surface, caused by low vibration or other small external forces, such as slight jumping accompanied by movement within a medical institution, dropping from a low position during placement on a stand such as a table, and tapping, which are often applied to the prefilled syringe package on a daily basis.

### Solution to Problem

The present inventors have conducted extensive studies in order to solve the above-described problems.

As a result, it has been found that, in a prefilled syringe package obtained by packaging a prefilled syringe with a packaging material, by disposing a buffering material which is deformed by low vibration or other small external forces which are often applied to the prefilled syringe package on a daily basis and buffers the external forces in the packaging material together with the prefilled syringe, or by accommodating a prefilled syringe in a package which is deformed by low vibration or other small external forces applied to the prefilled syringe package to have an action for buffering the external forces, or by combining the two, it is possible to more favorably prevent damage to a prefilled syringe or liquid leakage in a case where a large external force such as vibration during dropping from a high position or transporting is applied to a prefilled syringe package, and to prevent or reduce problems such as aggregation of components in a drug solution with which a syringe is filled, falling off of a liquid lubricant such as silicone oil applied to an inner wall surface of a syringe main body from the wall surface, and aggregation of components contained in a drug solution in a syringe due to a lubricant such as silicone oil fallen off from a wall surface in a case where low vibration or other weak external forces are applied to the prefilled syringe package.

That is, the present invention is directed to
(1) a prefilled syringe package including: a prefilled syringe prefilled with a liquid; and a packaging material in which the prefilled syringe is accommodated, the prefilled syringe package being any one of
   (A) a prefilled syringe package in which a buffering material for buffering an external force by being deformed by the external force is disposed between the prefilled syringe in the packaging material and the packaging material, (B) a prefilled syringe package in which the prefilled syringe is accommodated in the packaging material which includes a support portion for supporting the prefilled syringe accommodated in the packaging material and a buffer portion which is disposed further on the outside of the support portion while being formed continuously with the support portion and has an action for buffering an external force by being deformed by the external force, and (C) a prefilled syringe package in which the prefilled syringe is accommodated in the packaging material, which includes a support portion for supporting the prefilled syringe accommodated in the packaging material and a buffer portion which is disposed further on the outside of the support portion and has an action for buffering an external force by being deformed by the external force, and a buffering material for buffering an external force by being deformed by the external force is disposed between the prefilled syringe in the packaging material and the packaging material.

Moreover, the present invention is directed to
(2) the prefilled syringe package according to (1), in which the buffering material is one or more kinds selected from an impact absorbing gel, a polyethylene foam buffering material, soft organic polymer foam, an elastic body, and a fiber aggregate, and
(3) the prefilled syringe package according to (1) or (2), in which the packaging material of the prefilled syringe packages of (B) and (C) is either a packaging material having one or more of a bellows-like structure, a spring-like structure, and a sponge-like structure which are deformed by an external force as the buffer portion, or a packaging material made of a material having an action for buffering an external force by being deformed by the external force.

Furthermore, the present invention is directed to
(4) the prefilled syringe package according to any one of (1) to (3), in which the package is a form of blister packaging, and
(5) the prefilled syringe package according to any one of (1) to (4), in which the prefilled syringe includes a
syringe main body filled with the liquid and a gasket slidable within the syringe main body, and silicone oil is applied on an inner wall surface of the syringe main body and/or a surface of the gasket.

Moreover, the present invention is directed to
(6) the prefilled syringe package according to any one of (1) to (5), in which the liquid with which the prefilled syringe is prefilled is water for injection, a liquid containing a proteinaceous substance, a peptide, a nucleic acid, or a conjugate vaccine, and
(7) the prefilled syringe package according to any one of (1) to (6), in which the liquid with which the prefilled syringe is prefilled is a liquid containing biopharmaceuticals.

Furthermore, the present invention is directed to
(8) a drug kit including: the prefilled syringe package of (5), and a drug container in which a drug to be diluted with or dissolved in the liquid is accommodated.

### Advantageous Effects of Invention

The prefilled syringe package of the present invention not only can more favorably prevent damage to or liquid leakage from a prefilled syringe when a large external force, such as vibration during dropping from a high position or transporting, is applied to the prefilled syringe package, but also can prevent or reduce occurrence of aggregation of components in a drug solution with which a syringe is filled, falling off of a lubricant such as silicone oil applied to an inner wall surface of a syringe main body from the wall surface, and aggregation of components contained in a drug solution in the syringe due to a lubricant such as silicone oil fallen off from a wall surface, in a case where low vibration or other weak external forces, such as slight jumping accompanied by movement within a medical institution, dropping from a low position during placement on a stand such as a table, and tapping, which are often applied to the prefilled syringe package on a daily basis are applied to the prefilled syringe package. For this reason, problems such as a decrease in drug efficacy of a drug with which a syringe is filled, a side effect due to aggregated components, and a decrease in a drug efficacy accompanied by production of antibodies against aggregated components do not occur. In addition, in a case where a liquid containing silicone oil fallen off from a wall surface is mixed with a drug, problems such as a decrease in drug efficacy of the drug mixture, a side effect due to aggregated components, and a decrease in drug efficacy accompanied by production of antibodies against aggregated components do not occur.

A method for applying silicone oil as a lubricant to an inner wall surface of a syringe main body is widely performed in order to reduce sliding resistance of a gasket in a syringe. However, it is known that silicone oil fallen off from the inner wall surface of the syringe main body causes a side effect or a decrease in drug efficacy by aggregating proteins in a protein-containing drug solution with which the syringe is filled (PTL 5) . In addition, even in the case where a liquid containing silicone oil fallen off from a wall surface is mixed with a drug, there is a concern that a side effect or a decrease in drug efficacy of the drug mixture may be caused.

In the prefilled syringe package of the present invention in which silicone oil is applied to an inner wall surface of a syringe main body, it is possible to prevent or reduce falling off of the silicone oil from the inner wall surface when low vibration or other small external forces are applied as described above. Therefore, it is possible to prevent aggregation or the like of proteins accompanied by the falling off of the silicone oil.

Furthermore, in the prefilled syringe package of the present invention that can reduce vibration or other external forces applied to the prefilled syringe, even in a case where a liquid with which the syringe is filled with contains medicinal components such as a conjugate vaccine, a nucleic acid, and a peptide such as octreotide which are known to be easily aggregated by vibration or the like, it is possible to prevent the decrease in drug efficacy, the side effect, or the like by preventing or reducing the aggregation of the medicinal components. In addition, even in a case where a liquid with which the syringe is filled is mixed with a drug having the medicinal components, it is possible to prevent the decrease in drug efficacy, the side effect, or the like by preventing or reducing the aggregation of the medicinal components.

### Brief Description of Drawings

[Fig. 1] Fig. 1 is a schematic diagram showing an example of a prefilled syringe package of an aspect (A) of the present invention.
[Fig. 2] Fig. 2 is a schematic diagram showing an example of a prefilled syringe package of an aspect (B) of the present invention.
[Fig. 3] Fig. 3 is a schematic diagram showing another example of the prefilled syringe package of the aspect (B) of the present invention.
[Fig. 4] Fig. 4 is a schematic diagram showing still another example of the prefilled syringe package of the aspect (B) of the present invention.

### Description of Embodiments

Hereinafter, the present invention will be described in detail.

A prefilled syringe package of the present invention obtained by packaging a prefilled syringe in which a liquid is prefilled with a packaging material is any one of (A) a prefilled syringe package in which a buffering material for buffering an external force by being deformed by the external force is disposed between the prefilled syringe in the packaging material and the packaging material, (B) a prefilled syringe package in which the prefilled syringe is accommodated in the packaging material which includes a support portion for supporting the prefilled syringe accommodated in the packaging material and a buffer portion which is disposed further on the outside of the support portion while being formed continuously with the support portion and has an action for buffering an external force by being deformed by the external force, or (C) a prefilled syringe package in which the prefilled syringe is accommodated in the packaging material, which includes a support portion for supporting the prefilled syringe accommodated in the packaging material and a buffer portion which is disposed further on the outside of the support portion and has an action for buffering an external force by being deformed by the external force, and a buffering material for buffering an external force by being deformed by the external force is disposed between the prefilled syringe in the packaging material and the packaging material.

Here, the "buffering material for buffering an external force applied to the prefilled syringe package by being deformed by the external force" of the present invention refers to a buffering material having an action for buffering (alleviating, reducing, or eliminating) the external force applied to the prefilled syringe by temporarily deforming the buffering body itself or maintaining the deformed state due to the applied external force when a large external force is applied to the prefilled syringe due to vibration or the like during dropping of the prefilled syringe package from a high position or transporting the prefilled syringe package, or when a small external force is applied to the prefilled syringe due to slight jumping or the like accompanied by movement of the prefilled syringe package within a medical institution, dropping from a low position during placement of the prefilled syringe package on a stand such as a table, and tapping.

In addition, the "buffer portion which has an action for buffering an external force by being deformed by the external force" of the present invention, which is included in the packaging material, refers to a structure having an action for buffering (alleviating, reducing, or eliminating) the external force applied to the prefilled syringe by temporarily deforming the buffer portion or maintaining the deformed state due to the applied external force when a large external force is applied to the prefilled syringe package due to vibration or the like during dropping of the prefilled syringe package from a high position or transporting the prefilled syringe package, or when a small external force is applied to the prefilled syringe due to slight jumping or the like accompanied by movement of the prefilled syringe package within a medical institution, dropping from a low position during placement of the prefilled syringe package on a stand such as a table, and tapping.

Although there is no restriction, the prefilled syringe package of the present invention will be described with reference to the drawings.

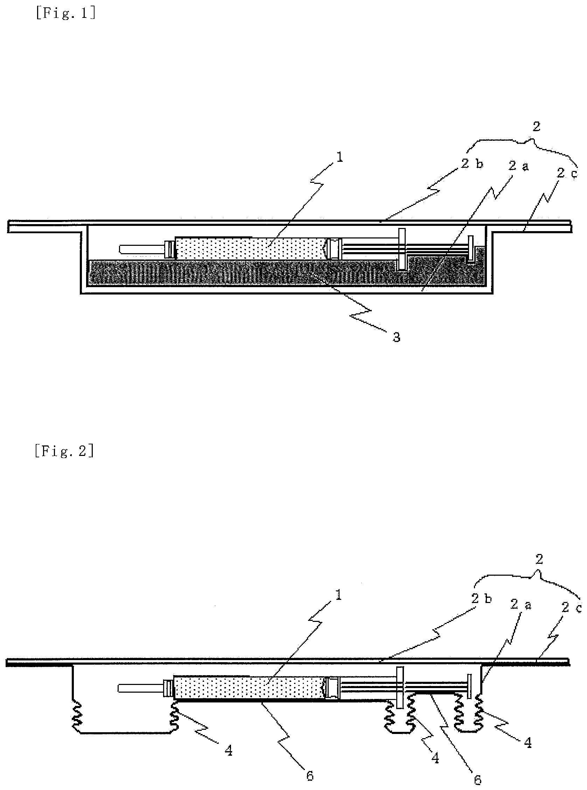

Fig. 1 is a schematic diagram showing an example of a prefilled syringe package of the above-described aspect (A) and is a longitudinal cross-sectional view following a longitudinal direction of the prefilled syringe.

In the prefilled syringe package of Fig. 1, a prefilled syringe 1 is accommodated in a packaging material 2 (a space between a packaging material main body 2a and a lid body 2b), a buffering material 3 for buffering an external force applied to the prefilled syringe package by being deformed by the external force is disposed between the prefilled syringe 1 and the packaging material 2 in the packaging material main body 2a, and the prefilled syringe package is sealed by the lid body 2b.

In the prefilled syringe package of the aspect (A) shown in Fig. 1, it is possible to prevent occurrence of a large accident such as damage to a prefilled syringe 1 or liquid leakage by buffering (alleviating, reducing, or eliminating) a large external force applied to the prefilled syringe 1 using the buffering material 3 deformed by the large external force applied to the prefilled syringe 1 due to vibration or the like during dropping of the prefilled syringe package from a high position or transporting. In addition, it is possible to prevent or reduce occurrence such as aggregation of components in a drug solution accommodated in a syringe, falling off of silicone oil applied to an inner wall surface of a syringe main body from the wall surface, and aggregation of proteinaceous substances or other components contained in a drug solution in the syringe due to silicone oil fallen off from a wall surface, by buffering external forces applied to the prefilled syringe 1 using the buffering material 3 deformed even by small vibration or other small external forces, such as slight jumping accompanied by movement of the prefilled syringe package within a medical institution, dropping from a low position during placement on a stand such as a table, and tapping, which are often applied to the prefilled syringe package on a daily basis.

It is possible to use any buffering material as the buffering material 3 used in the prefilled syringe package of the aspect (A) as long as it has an action for buffering an external force applied to the prefilled syringe package by being deformed by the external force, has excellent safety, hygienic properties, handling properties, and the like, and can be disposed in the packaging material 2, and specific examples thereof include impact absorbing gel, a polyethylene foam buffering material, soft organic polymer foam, an elastic body, and a fiber aggregate. One or more kinds thereof can be used.

It is possible to select a suitable one from gel having impact absorbing performance as the impact absorbing gel. Although there is no restriction, examples thereof include urethane gel, silicone gel, and water-containing gel of a superabsorbent resin such as sodium polyacrylate.

The polyethylene foam buffering material is a buffering material which consists of two polyethylene sheets and in which a large number of projections are formed on one or both polyethylene sheets and the space formed by the projections is enclosed by air for sealing. The polyethylene foam buffering material is sold under trade names of "AIR CAP", "PUTI PUTI" (registered trademark), "MINAPACK", "AIR PACKING", "AIR CUSHION", "AIR MATTRESS", "CAPRON", and "AIRBAG" depending on manufacturers. In the present invention, it is possible to select one suitable for each situation from polyethylene foam buffering materials well known in the related art.

Various soft organic polymer foams, for example, soft polyurethane foam, polyethylene sponge, rubber sponge, and thermoplastic elastomer sponge are known as the soft organic polymer foam, and it is possible to use one suitable for each situation from the soft organic polymer foam well known in the related art.

Examples of the elastic body include natural rubber, various synthetic rubbers, and a thermoplastic elastomer, and one or more kinds thereof can be used.

Examples of the fiber aggregate include a nonwoven pulp aggregate, a nonwoven fiber aggregate consisting of one or more kinds of natural fibers and synthetic fibers.

In the prefilled syringe package of the aspect (A) of the present invention, when disposing the buffering material 3 between the prefilled syringe 1 and the packaging material 2 in the packaging material, it is possible to appropriately select the disposition position or disposition state of the buffering material in the packaging material depending on the shape, structure, and size of the prefilled syringe, the type, shape, structure, and size of the packaging material, the type, shape, and size of the buffering material, and the type or physical properties of a liquid with which the syringe is filled so as to obtain a more favorable buffering action while considering costs or the like.

For example, the buffering material may be disposed so that the entire space within the packaging material is filled with the buffering material so as to surround the prefilled syringe, may be disposed in one spot in the space in the packaging material, or may be disposed in a plurality of spots in the space in the packaging material.

A case where the buffering material 3 is disposed in the lower portion of the prefilled syringe 1 in the packaging material 2 is exemplified in Fig. 1.

In the prefilled syringe package of the aspect (B) of the present invention, a packaging material which does not require a large amount of labor or cost for its manufacture is preferably used as the packaging material having a buffer portion, which has an action for buffering an external force applied to the prefilled syringe package by being deformed by the external force, further on the outside of a support portion. Examples thereof include a packaging material having a buffer portion including one or more of a bellows-like structure, a spring-like structure, and a sponge-like structure which are deformed by an external force to buffer the external force further on the outside of a support portion for supporting the prefilled syringe 1. In this case, the packaging material itself may be made of a material having an action for buffering an external force by being deformed by the external force.

Although there is no restriction, specific examples of the prefilled syringe package of the aspect (B) include those shown in the schematic diagrams of Figs. 2 to 4.

Fig. 2 is an example of the prefilled syringe package of which the prefilled syringe 1 having a support portion 6 for supporting the prefilled syringe 1 and a buffer portion 4 having bellows-like structures at three spots in the lower portion (bottom portion) positioned further on the outside of the support portion 6 is accommodated in the packaging material main body 2a and which is sealed with the lid body 2b, and is a longitudinal cross-sectional view following the longitudinal direction of the prefilled syringe.

In the prefilled syringe package of Fig. 2, a large accident such as damage to the prefilled syringe 1 or liquid leakage is prevented by buffering a large external force such as vibration during dropping from a high position or transporting through deformation (expansion and contraction) of the buffer portion 4 with the bellows-like structure formed in the lower portion of the packaging material main body 2a when the external force is received. In addition, external forces applied to the prefilled syringe package is buffered through deformation (expansion and contraction) of the buffer portion 4 with the bellows-like structure even by small vibration or other small external forces, such as slight jumping accompanied by movement of the prefilled syringe package within a medical institution, dropping from a low position during placement on a stand such as a table, and tapping, which are often applied to the prefilled syringe package on a daily basis. For this reason, the external forces are hardly transmitted to the prefilled syringe 1 supported by the support portion 6. For this reason, it is possible to prevent or reduce occurrence such as aggregation of medicinal components in a drug solution with which the prefilled syringe 1 is filled, falling off of silicone oil applied to an inner wall surface of a syringe main body of the prefilled syringe 1 from the wall surface, and aggregation of proteinaceous substances or other components contained in a drug solution in the prefilled syringe 1 due to silicone oil fallen off from a wall surface.

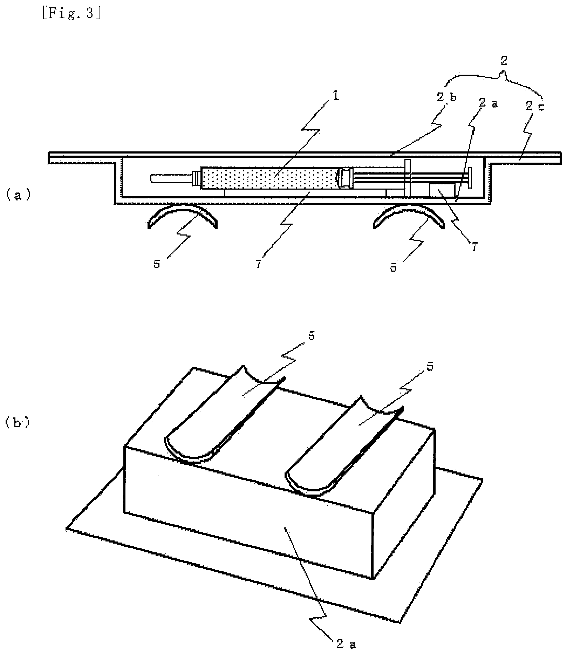

Fig. 3 is a schematic diagram of an example of the prefilled syringe package in which a buffer portion 5 having a gutter-like, leaf spring-like structure having a circular arc-like cross section is provided on an outer bottom portion of the packaging material main body 2a and the prefilled syringe 1 is accommodated in the packaging material 2. (a) of Fig. 3 is a longitudinal cross-sectional view following the longitudinal direction of the prefilled syringe and (b) of Fig. 3 is a schematic diagram in a case where the outer bottom surface of the packaging material main body 2a is viewed from the outside. A support portion 7 for supporting the prefilled syringe 1 is provided in the bottom portion of the packaging material main body 2a and the buffer portion 5 having the leaf spring-like structure is positioned further on the outside of the support portion 7.

In the prefilled syringe package of Fig. 3, it is possible to prevent a large accident such as damage to the prefilled syringe 1 or liquid leakage by buffering a large external force, such as vibration during dropping from a high position or transporting, through elastic deformation of the buffer portion 5 having the leaf spring-like structure provided on the outer bottom surface of the packaging material main body 2a when the large external force is received. In addition, it is possible to prevent or reduce occurrence such as aggregation of medicinal components in a drug solution accommodated in a syringe, falling off of silicone oil applied to an inner wall surface of a syringe main body from the wall surface, and aggregation of proteinaceous substances or other components contained in a drug solution in the syringe due to silicone oil fallen off from a wall surface since external forces applied to the prefilled syringe 1 is buffered using the buffer portion 5 which has the leaf spring-like structure and is elastically deformed even by small vibration or other small external forces, such as slight jumping accompanied by movement of the prefilled syringe package within a medical institution, dropping from a low position during placement on a stand such as a table, and tapping, which are often applied to the prefilled syringe package on a daily basis.

In the prefilled syringe package of Fig. 3, the buffer portion 5 having the leaf spring-like structure is preferably made of a material, particularly plastic and/or metal, which exhibits leaf spring characteristics.

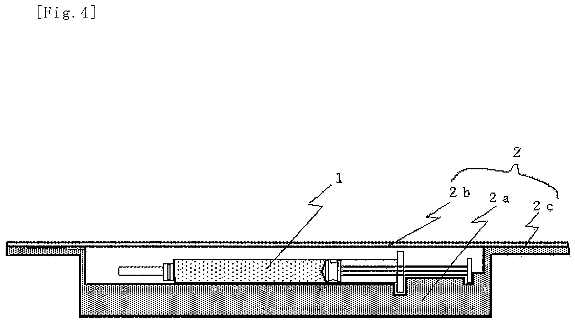

Fig. 4 is a schematic diagram of an example of a prefilled syringe package of which the packaging material main body 2a itself of the packaging material is made of a material having an action for alleviating an external force applied to the prefilled syringe package by being deformed by the external force. In the prefilled syringe package of Fig. 4, a portion in the bottom portion of the packaging material main body 2a which abuts on the prefilled syringe 1 is a support portion for supporting the prefilled syringe 1 and a portion positioned lower than the support portion corresponds to a buffer portion. That is, the bottom portion of the packaging material main body 2a includes the support portion facing the prefilled syringe 1 and the buffer portion positioned outside the support portion. In Fig. 4, examples of the "material having an action for alleviating an external force by being deformed by the external force" which forms the packaging material main body 2a include soft organic polymer foam such as soft polyurethane foam, polyethylene sponge, rubber sponge, and thermoplastic elastomer sponge, elastic bodies such as natural rubber, various synthetic rubbers, and a thermoplastic elastomer, a nonwoven pulp aggregate, and a nonwoven fiber aggregate consisting of one or more kinds of natural fibers and synthetic fibers, and the packaging material main body 2a can be made of one or more kinds of the materials.

In the prefilled syringe package of Fig. 4, it is possible to prevent a large accident such as damage to the prefilled syringe 1 or liquid leakage using buffering characteristics of the packaging material main body 2a itself when a large external force, such as vibration during dropping from a high position or transporting. In addition, it is possible to prevent or reduce occurrence such as aggregation of medicinal components in a drug solution accommodated in a syringe, falling off of silicone oil applied to an inner wall surface of a syringe main body from the wall surface, and aggregation of proteinaceous substances or other components contained in a drug solution in the syringe due to silicone oil fallen off from a wall surface since the external force applied to the prefilled syringe 1 is buffered through a buffering action of the packaging material main body 2a even by small vibration or other small external forces, such as slight jumping accompanied by movement of the prefilled syringe package within a medical institution, dropping from a low position during placement on a stand such as a table, and tapping, which are often applied to the prefilled syringe package on a daily basis.

Although not described in Figs. 1 to 4, it is desirable that a fixing unit or fixing means (for example, a clamping portion or a projection) for preventing the prefilled syringe 1 accommodated in the packaging material from moving in the packaging material is integrally provided with the packaging material 2 (the packaging material main body 2a and/or the lid body 2b) or provided as a separate member in the prefilled syringe packages of the aspects of (A) to (C). Furthermore, it is desirable that a fixing unit or fixing means (for example, a clamping portion or a projection) for fixing the buffering material 3 is integrally provided with the packaging material 2 (the packaging material main body 2a and/or the lid body 2b) or provided as a separate member in the prefilled syringe packages of the aspects of (A) and (C) in order to prevent the buffering material 3 disposed in the packaging material from moving in the packaging material. Alternately, it is desirable that the buffering material 3 is bonded to the packaging material 2 using an adhesive or a gluing agent for fixation.

Although not shown in the drawings, another example of the prefilled syringe package of the aspect (B) includes a prefilled syringe package obtained by accommodating a prefilled syringe in a packaging bag or the like produced using a material, such as polyethylene foam buffering material or nonwoven fiber aggregate, which shows a buffering action by being deformed by an external force.

The prefilled syringe package of the aspect (C) of the present invention is a combination of an appropriate one of the above-described aspect (A) and an appropriate one of the above-described aspect (B).

Although the type of the packaging material for accommodating the prefilled syringe is not particularly limited in the prefilled syringe packages of (A) to (C) of the present invention, it is possible to employ all of the packaging materials employed for packaging a prefilled syringe in the related art.

Examples of the packaging material used for the prefilled syringe package of the present invention include a packaging material for blister packaging, a bag-like packaging material, and a box-like packaging material.

Among these, it is possible to blister-package a prefilled syringe using a packaging material for blister packaging. In the case where a prefilled syringe is blister-packaged, it is possible to prevent or reduce damage to a prefilled syringe, liquid leakage, and the like in a case where a large external force such as vibration during dropping from a high position or transporting is received.

When blister packaging a prefilled syringe, it is possible to employ a packaging material and a blister packaging form which has been used for blister packaging of a syringe or a prefilled syringe in the related art.

A syringe (injector) includes, in general, a syringe main body (injection cylinder, outer cylinder), a gasket disposed in the syringe main body, a pusher (plunger) for moving (sliding) the gasket in the syringe main body, and an injection needle attachment portion and an injection needle which are provided at a distal end in the other end portion of the syringe main body.

The "prefilled syringe in which a liquid is accommodated" in the prefilled syringe package of the present invention refers to a syringe of which a syringe main body is prefilled with a liquid so as to prevent the liquid from leaking outside.

In the prefilled syringe package of the present invention, any shape, structure, and dimension of the entire syringe and each portion may be used and any material (a material of the syringe main body, the gasket, the pusher, the injection needle attachment portion, the injection needle, a seal cap attached to a distal end of the syringe, and the like) of each portion of the syringe may be used.

Although there is no restriction, in the present invention, the syringe main body may be made of glass or plastic. Examples of plastic forming the syringe main body include polyethylene, polypropylene, a cyclic olefin polymer, polyolefin such as poly-(4-methylpentene-1), polycarbonate, polyester such as polyethylene terephthalate and polyethylene naphthalate, polyamide, and polyvinyl chloride. The syringe main body can be made of one or more kinds of the plastic.

Among these, the syringe main body is preferably made of a cyclic olefin polymer in the present invention from the viewpoint of breakage resistance, handling properties, lightness, transparency, and low elution properties.

The syringe main body (injection cylinder) may have a single-chamber shape or a multi-chamber shape which is divided into multiple chambers. In the syringe main body having the multi-chamber shape, a drug is accommodated in one chamber and a liquid (such as a dissolution liquid, a dilution liquid, and a dispersion liquid for the drug) is accommodated in a separate chamber. The drug is mixed with the liquid by allowing communication between the both chambers when in use. The drug is dissolved or dispersed in or diluted with the liquid and is discharged out of the syringe in the liquid state.

In the present invention, the gasket of the syringe is made of an elastic body which has excellent liquid sealing properties and has no problem in terms of safety, hygienic properties, and the like.

Specific examples of the elastic body for the gasket include styrene thermoplastic elastomers such as a styrene-isoprene block copolymer or a hydrogenated product thereof, and a styrene-butadiene block copolymer or a hydrogenated product thereof, olefin thermoplastic elastomers such as an ethylene-α-olefin copolymer, polyvinyl chloride thermoplastic elastomers, polyester thermoplastic elastomers, polyamide thermoplastic elastomers, polyurethane thermoplastic elastomers, butyl rubber, halogenated butyl rubber, isoprene rubber, chloroprene rubber, butadiene rubber, nitrile-butadiene rubber, styrene-butadiene rubber, natural rubber, and silicone rubber. The gasket can be made of one or more kinds of these elastic bodies.

Among these, the gasket is preferably made of butyl rubber from the viewpoint of liquid sealing properties, sliding properties in the syringe main body, hygienic properties, low elution properties, and the like.

In the present invention, an injection needle may be previously attached to the syringe main body in the prefilled syringe package. Alternately, an injection needle may be designed to be easily attached to a distal end of the syringe main body without being previously attached thereto.

In the prefilled syringe package of the present invention, the type of liquid with which the syringe main body is prefilled is not particularly limited. Examples thereof include a liquid for dissolving or dispersing a drug such as water for injection or physiological saline therein or for diluting the drug therewith, a solution in which a drug is dissolved, and a dispersion in which a drug is dispersed.

Examples of the drug with which the syringe main body can be prefilled include a proteinaceous substance, a peptide, a nucleic acid, and a conjugate vaccine.

In the present invention, specific examples of the proteinaceous substance with which a syringe can be prefilled include monoclonal antibodies, a granular colony-stimulating factor, erythropoietin, interferon, interleukin, insulin, growth hormones, a tissue plasminogen activation factor, thrombopoietin, urokinase, serum albumin, a blood coagulation factor VIII, leptin, a stem cell growth factor, etanercept, a recombinant protein vaccine, and a purified protein vaccine. The proteinaceous substances may be produced by applying biotechnology such as gene recombination, cell fusion, and cell culture, or may be produced through other methods.

In the prefilled syringe package of the present invention, the liquid with which the syringe main body is prefilled may contain one or more kinds of a solubilizing agent, an isotonic agent, a pH adjuster, a buffer agent, a reducing agent, an antioxidant, and a surfactant (particularly a nonionic surfactant) as necessary.

In the prefilled syringe package of the present invention, any one or both of the inner wall surface of the syringe main body and the surface of the gasket may be coated or covered with a lubricant for making movement (sliding) of the gasket in the syringe main body smooth, or may not be coated or covered with the lubricant. At least the inner wall surface of the syringe main body is preferably coated or covered with a lubricant for smooth sliding of the gasket.

An example of the lubricant with which the inner wall surface of the syringe main body is coated or covered includes silicone oil. In general, silicone oil is applied to the inner wall surface of the syringe main body in the syringe.

Silicone oil having a viscosity of 20 to 20,000 cSt, more preferably 1,000 to 15,000 cSt, particularly preferably 10,000 to 13,000 cSt at normal temperature (20°C) is preferably used as the silicone oil to be applied to the inner wall surface of the syringe main body. Silicone oil having a viscosity of 10,000 to 13,000 cSt at normal temperature (20°C) is easily applied to the inner wall surface of the syringe main body and is hardly fallen off from the inner wall surface of the syringe main body.

Examples of the silicone oil include diorganopolysiloxane oils such as dimethylpolysiloxane oil and methylphenylpolysiloxane oil. Among these, dimethylpolysiloxane oil or a composition thereof is preferably used.

As described above, it has been found through the studies of the present inventors that silicone oil applied to the inner wall surface of the syringe main body is easily fallen off from the inner wall surface of the syringe main body not only in a case where a large external force, such as vibration during dropping of the prefilled syringe package from a high position or transporting the prefilled syringe package, is applied to the prefilled syringe, but also in a case where a small external forces, such as slight jumping accompanied by movement of the prefilled syringe package within a medical institution, dropping from a low position during placement of the prefilled syringe package on a stand such as a table, and tapping, are applied to the prefilled syringe.

Silicone oil fallen off from the inner wall surface of the syringe main body causes aggregation of a drug in a liquid with which the syringe is filled, particularly a drug consisting of a proteinaceous substance and a peptide, and causes various harmful phenomena such as a decrease in drug efficacy or a side effect.

In a case of employing any one of the above-described aspects (A) to (C) in the prefilled syringe package of the present invention, it is possible to prevent a large accident such as damage to the prefilled syringe or liquid leakage when a large external force, such as vibration during dropping from a high position or transporting, is applied to the prefilled syringe, and to prevent or reduce falling off of silicone oil from the wall surface of the syringe main body caused even by small vibration or other small external forces, such as slight jumping accompanied by movement of the prefilled syringe package within a medical institution, dropping from a low position during placement on a stand such as a table, and tapping, which are often applied to the prefilled syringe package on a daily basis. For this reason, it is possible to prevent or reduce aggregation of a drug in a liquid accommodated in a syringe due to fallen off silicone oil, particularly a drug such as a proteinaceous substance, a peptide, a nucleic acid, or a conjugate vaccine.

In addition, a liquid with which the syringe main body, of which the inner wall surface is coated with silicone oil, is prefilled may be a liquid for dissolving or dispersing a drug such as a proteinaceous substance accommodated in a container such as a vial therein or for diluting the drug therewith. Examples of such a liquid include water for injection and physiological saline. The prefilled syringe package including the prefilled syringe prefilled the liquids may be provided in a form of a drug kit together with a drug container such as a vial accommodating a drug to be dissolved and dispersed in the liquids or diluted with the liquids. Even in such a drug kit, it is possible to prevent or reduce fallen off of silicone oil from the inner wall surface of the syringe main body due to a small external force applied to the prefilled syringe package. For this reason, in a case where a drug accommodated in a drug container such as a vial is dissolved, diluted, or dispersed, it is possible to prevent or reduce aggregation of the drug accommodated in a drug container, particularly a drug such as a proteinaceous substance, a peptide, a nucleic acid, and a conjugate vaccine due to fallen off silicone oil, using the prefilled syringe of the drug kit.

### Examples

Hereinafter, the present invention will be described in more detail with reference to examples or the like, but is not limited to the following examples.

### <<Example 1>> [Manufacture of Prefilled Syringe Package in which Buffering Material is Accommodated in Packaging Material]

### (1) Manufacture of Prefilled Syringe:

(i) A syringe main body (outer cylinder) [wall thickness = 1.0 mm, inner diameter = 6.3 mm, length from distal end (injection needle attachment portion) to rear end opening portion = 64.3 mm] having the shape shown in Fig. 1 was manufactured using glass. Silicone oil [dimethylpolysiloxane oil, viscosity = 12,500 cSt (mm²/s) (20°C)] was applied to the entire inner wall surface of the syringe main body in an amount of 0.2 mg per syringe.
(ii) A 1 mL capacity syringe was prepared using the syringe main body which was obtained in the above-described (i) and of which the entire inner wall surface was coated with silicone oil, a plunger obtained by attaching a gasket (made of butyl rubber) having the shape shown in Fig. 1 to a distal end of the plunger, and a seal cap for sealing a distal end of the syringe main body.
(iii) The syringe prepared in the above-described (ii) was filled with 1 mL of water for injection, the distal end of the syringe main body was sealed with the seal cap, and the gasket-attached plunger was inserted into the syringe main body from its rear end to manufacture the prefilled syringe 1 which had the structure shown in Fig. 1 and of which the syringe main body is filled with the water for injection.

### (2) Manufacture of Prefilled Syringe Package

(i) A packaging material main body 2a (tray type container) for blister packaging which had the shape shown in Fig. 1 and had a concave portion having a rectangular parallelepiped shape and a flange portion 2c surrounding the periphery of an upper end of the concave portion was manufactured (vertical dimension of concave portion with rectangular parallelepiped shape = 130 mm, horizontal dimension = 30 mm, depth = 15 mm, and width of flange portion 2c = 5 mm) through vacuum molding using a polypropylene sheet.
(ii) A buffering material 3 formed of a small bag (15 g of filling amount of water-containing gel) filled with impact absorbing gel [water-containing gel (water content = 95 mass%) of sodium polyacrylate; manufactured by Wako Pure Chemical Industries, Ltd.] was disposed in the concave portion of the packaging material main body 2a for blister packaging manufactured in the above-described (i) as shown in Fig. 1, and then, a prefilled syringe 1 which was filled with water for injection and manufactured in the above-described (1) was placed on the buffering material so as to be substantially horizontal. At that time, a flange portion around the rear end opening portion of the syringe main body of the prefilled syringe 1 and a part of a disk portion at a rear end of a plunger were pushed into and embedded in the buffering material 3 as shown in Fig. 1 in order to make the prefilled syringe 1 substantially horizontal.
(iii) The prefilled syringe 1 and the buffering material 3 accommodated in the packaging material main body 2a were fixed to the packaging material main body 2a using adhesive tape so as not to move, and then, the packaging material main body 2a was covered with a lid body 2b made of polyethylene terephthalate and was sealed with a flange portion 2c to manufacture a prefilled syringe package.

### <<Example 2>> [Manufacture of Prefilled Syringe Package in which Packaging Material Having Buffer Portion is Used]

### (1) Manufacture of Packaging Material:

A packaging material main body 2a for blister packaging which integrally had a buffer portion 4 (bellows-like leg portion) having a bellows-like structure extending along a width direction of the packaging material main body 2a at three spots in a bottom portion of a rectangular parallelepiped concave portion of the packaging material main body 2a having the shape shown in Fig. 2 was manufactured (vertical dimension of concave portion with rectangular parallelepiped shape = 145 mm, horizontal dimension = 30 mm, depth = 20 mm, width of flange portion = 5 mm, and height dimension of buffer portion 4 = 10 mm) through vacuum molding using a polypropylene sheet.

### (2) Manufacture of Prefilled Syringe Package

The prefilled syringe 1 with water for injection which was manufactured in the same manner as in (1) of Example 1 was disposed in the concave portion of the packaging material main body 2a for blister packaging manufactured in the above-described (1) and was fixed to the packaging material main body 2a using adhesive tape so as not to move. Then, the packaging material main body 2a was covered with a lid body 2b made of polyethylene terephthalate and was sealed with a flange portion 2c to manufacture a prefilled syringe package.

### <<Comparative Example 1>>

The prefilled syringe 1 with water for injection which was manufactured in the same manner as in (1) of Example 1 was directly disposed in a concave portion of the packaging material main body 2a for blister packaging manufactured in the same manner as in (i) of (2) of Example 1 as it is without disposing a buffering material, and was fixed to the packaging material main body 2a using adhesive tape so as not to move. Then, the packaging material main body 2a was covered with a lid body 2b made of polyethylene terephthalate and was sealed with a flange portion 2c to manufacture a prefilled syringe package.

### <<Test Example 1>>

(1) A rotating drop test was performed on the prefilled syringe packages obtained in Examples 1 and 2 and Comparative Example 1 and an unpackaged prefilled syringe [the same prefilled syringe obtained in (1) of Example 1] using a tablet friability tester specified in ICH Q4B Annex 9 to examine the fallen state of silicone oil applied to the inner wall surface of each syringe from the wall surface.

Specifically, a "tablet friability tester OSK97NI132" manufactured by AS ONE Corporation was used as a friability tester, any one of the prefilled syringe packages obtained in Examples 1 and 2 and Comparative Example 1 or the unpackaged prefilled syringe [the same prefilled syringe obtained in (1) of Example 1] was accommodated in a drum of the friability tester. The prefilled syringe packages or the prefilled syringe were rotated and dropped within the drum under the conditions of a rotational speed of the drum of 100 rpm, a temperature of 25°C, and a number of rotating drops of 50, and were then taken out.

Next, the water for injection was taken out from each of the prefilled syringes, and the number of silicone oil fine particles dispersed in the water for injection was measured using a flow cytoparticle image analyzer ["FlowCam" (registered trademark) manufactured by Flow Imaging Technologies, Inc.]. During the measurement, the number of clear circular particles having an aspect ratio (minor axis length ÷ major axis length) of greater than or equal to 0.9 was counted as the silicone oil particles.

The results are shown in Table 1.

**[Table 1]**

| | Number of silicon oil fine particles (particles / mL) |
|---|---|
| Prefilled syringe package of Example 1 | 1,604 |
| Prefilled syringe package of Example 2 | 2,523 |
| Prefilled syringe package of Comparative Example 1 | 6,202 |
| Unpackaged prefilled syringe¹⁾ | 46,119 |

| | |
|---|---|
| 1) The same prefilled syringe manufactured in (1) of Example 1 | |

As can be seen from the results of Table 1, the number of silicone oil particles in the liquid for injection after the rotating drop test is performed is small and silicone oil hardly falls off from the inner wall surface of the syringe main body even if the external force is applied in the prefilled syringe package of Example 1 in which the buffering material for buffering an external force by being deformed by the external force is disposed between the prefilled syringe in the packaging material and the packaging material and in the prefilled syringe package of Example 2 in which the prefilled syringe is packaged using the packaging material in which the buffer portion having an action for buffering an external force by being deformed by the external force is disposed outward.

In contrast, in the prefilled syringe package of Comparative Example 1 in which the prefilled syringe is disposed without disposing a buffering material in the packaging material and which does not have an action for buffering an external force by being deformed by the external force, the number of silicone oil particles in the liquid for injection after the rotating drop test is performed is greatly larger than that in Examples 1 and 2 and silicone oil easily falls off from the inner wall surface of the syringe main body when the external force is applied.

In addition, the number of silicone oil particles in the liquid for injection after the rotating drop test is performed is significantly large and silicone oil very easily falls off from the inner wall surface of the syringe main body in the test in which the prefilled syringe which is not packaged with the packaging material is rotated and dropped as it is.

### <<Example 3>> [Manufacture of Prefilled Syringe Package in which Buffering Material is Accommodated in Packaging Material]

### (1) Manufacture of Prefilled Syringe:

(i) A syringe main body (outer cylinder) [wall thickness = 1.0 mm, inner diameter = 6.3 mm, length from distal end (injection needle attachment portion) to rear end opening portion = 64.3 mm] having the shape shown in Fig. 1 was manufactured using cyclic polyolefin (manufactured by Zeon Corporation).
(ii) A 1 mL capacity syringe was prepared using the syringe main body obtained in the above-described (i), a plunger obtained by attaching a gasket (made of butyl rubber) having the shape shown in Fig. 1 to a distal end of the plunger, and a seal cap for sealing a distal end of the syringe main body.
(iii) The syringe prepared in the above-described (ii) was filled with 1 mL of a protein preparation containing adalimumab, which was one type of monoclonal antibody, at a concentration of 1 mg/mL to manufacture a prefilled syringe 1 having the structure shown in Fig. 1.

### (2) Manufacture of Prefilled Syringe Package

(i) A packaging material main body 2a (tray type container) for blister packaging was manufactured (vertical dimension of concave portion with rectangular parallelepiped shape = 145 mm, horizontal dimension = 30 mm, depth = 15 mm, and width of flange portion 2c = 5 mm) using a polypropylene sheet in the same manner as in (i) of (2) of Example 1.
(ii) The same buffering material 3 as in (ii) of (2) of Example 1 was disposed in the concave portion of the packaging material main body 2a for blister packaging manufactured in the above-described (i) as shown in Fig. 1, and then, a prefilled syringe 1 which was filled with diluted HUMIRA and manufactured in the above-described (1) was placed on the buffering material so as to be substantially horizontal. At that time, a flange portion around the rear end opening portion of the syringe main body of the prefilled syringe and a part of a disk portion at a rear end of a plunger were embedded in the buffering material 3 as shown in Fig. 1 in order to make the prefilled syringe substantially horizontal.
(iii) The prefilled syringe 1 and the buffering material 3 accommodated in the packaging material main body 2a were fixed to the packaging material main body 2a using adhesive tape so as not to move, and then, the packaging material main body 2a was covered with a lid body 2b made of polyethylene terephthalate and was sealed with a flange portion 2c to manufacture a prefilled syringe package.

### <<Example 4>> [Manufacture of Prefilled Syringe Package in which Packaging Material Having Buffer Portion is Used]

### (1) Manufacture of Prefilled Syringe Package

A packaging material main body 2a for blister packaging which integrally had a buffer portion 4 (bellows-like leg portion) having a bellows-like structure extending along a width direction of the packaging material main body 2a at three spots in a bottom portion of a rectangular parallelepiped concave portion of the packaging material main body 2a was manufactured in the same manner as in (1) of Example 2.

(2) A diluted HUMIRA liquid-containing prefilled syringe 1 manufactured in the same manner as in (1) of Example 3 was disposed in the concave portion of the packaging material main body 2a for blister packaging manufactured in the above-described (1), and was fixed to the packaging material main body 2a using adhesive tape so as not to move. Then, the packaging material main body 2a was covered with a lid body 2b made of polyethylene terephthalate and was sealed with a flange portion 2c to manufacture a prefilled syringe package.

### <<Comparative Example 2>>

A diluted HUMIRA liquid-containing prefilled syringe 1 manufactured in the same manner as in (1) of Example 3 was directly disposed in a concave portion of the packaging material main body 2a for blister packaging manufactured in the same manner as in (i) of (2) of Example 3 as it is without disposing a buffering material, and was fixed to the packaging material main body 2a using adhesive tape so as not to move. Then, the packaging material main body 2a was covered with a lid body 2b made of polyethylene terephthalate and was sealed with a flange portion 2c to manufacture a prefilled syringe package.

### <<Test Example 3>>

The prefilled syringe packages obtained in Examples 3 and 4 and Comparative Example 2 and the diluted HUMIRA liquid-contained prefilled syringe (unpackaged prefilled syringe) manufactured in the same manner as in (1) of Example 3 were dropped 50 times from a height of 370 mm at room temperature (25°C).

Next, the diluted HUMIRA liquid was taken out from each prefilled syringe, and the number of protein aggregates in the liquid was measured using the same flow cytoparticle image analyzer used in Test Example 1. In the measurement, the detected particles were counted as the protein aggregates.

The occurrence rate (pieces / mg) of the protein aggregates was obtained according to Equation (1) from the number of protein aggregates (Prₐ) (pieces / mL) in the liquid after the drop test and the concentration of protein (Pr₀) (mg / mL) in the liquid before the drop test was performed. The results are as shown in Table 2.
[Equation 1] $Occurrence rate \left(pieces/mg\right) of protein aggregates = \left[{Pr}_{a}\left(pieces/mL\right)\div \left[{Pr}_{0}\left(mg/mL\right)\right]\right]$

**[Table 2]**

| | Occurrence rate of protein aggregates (pieces / mg) |
|---|---|
| Prefilled syringe package of Example 3 | 2,640 |
| Prefilled syringe package of Example 4 | 3,104 |
| Prefilled syringe package of Comparative Example 2 | 6,261 |
| Unpackaged prefilled syringe¹⁾ | 7,235 |

| | |
|---|---|
| 1) The same diluted HUMIRA liquid-containing prefilled syringe manufactured in (1) of Example 3 | |

As can be seen from the results of Table 2, falling off of silicone oil from the inner wall surface of the syringe main body occurs less frequently even if an external force is received in the prefilled syringe package of Example 3 in which a buffering material for buffering an external force by being deformed by the external force is disposed and in the prefilled syringe package of Example 4 in which a packaging material which has a buffer portion having an action for buffering an external force by being deformed by the external force outwardly is used. Therefore, the aggregation of proteins in the protein liquid (diluted HUMIRA liquid) with which the syringe is filled occurs less frequently.

In contrast, in a case where an external force is received in the prefilled syringe package of Comparative Example 2 in which the prefilled syringe is disposed without disposing a buffering material in the packaging material and which does not have an action for buffering an external force by being deformed by the external force and in the unpackaged prefilled syringe, falling off of silicone oil from the inner wall surface of the syringe main body occurs frequently. Therefore, the aggregation of proteins due to the fallen off silicone oil occurs frequently.

### Industrial Applicability

The prefilled syringe package of the present invention not only can more favorably prevent damage to or liquid leakage from a prefilled syringe when a large external force, such as vibration during dropping from a high position or transporting, is applied to the prefilled syringe package, but also can prevent or reduce occurrence of aggregation of components in a drug solution with which a syringe is filled, falling off of silicone oil applied to an inner wall surface of a syringe main body from the wall surface, and aggregation of components contained in a drug solution in the syringe due to silicone oil fallen off from a wall surface, in a case where low vibration or other weak external forces which are often applied to the prefilled syringe package on a daily basis are applied to the prefilled syringe package. For this reason, a decrease in drug efficacy of a drug with which a syringe is filled, a side effect due to aggregated components, a decrease in drug efficacy accompanied by production of antibodies against aggregated components, and the like do not occur. Moreover, even in a case where a liquid containing silicone oil fallen off from the wall surface is mixed with a drug, the problems such as the decrease in drug efficacy of the drug mixture, the side effect due to aggregated components, and the decrease in drug efficacy accompanied by production of antibodies against aggregated components does not occur. Therefore, the present invention is significantly useful in the medical field and the like.

The present application is based on Japanese Patent Application No. 2017-065820, filed March 29, 2017 and the disclosure thereof is incorporated as a whole by reference.

### Reference Signs List

- 1: prefilled syringe
- 2: packaging material
- 2a: packaging material main body
- 2b: lid body
- 2a: flange portion
- 3: buffering material
- 4: buffer portion having bellows-like structure
- 5: buffer portion having leaf spring-like structure
- 6: support portion
- 7: support portion

## Claims

1. A prefilled syringe package comprising:
a prefilled syringe prefilled with a liquid; and
a packaging material in which the prefilled syringe is accommodated,
the prefilled syringe package being any one of
(A) a prefilled syringe package in which a buffering material for buffering an external force by being deformed by the external force is disposed between the prefilled syringe in the packaging material and the packaging material,
(B) a prefilled syringe package in which the prefilled syringe is accommodated in the packaging material which includes a support portion for supporting the prefilled syringe accommodated in the packaging material and a buffer portion which is disposed further on the outside of the support portion while being formed continuously with the support portion and has an action for buffering an external force by being deformed by the external force, and
(C) a prefilled syringe package in which the prefilled syringe is accommodated in the packaging material, which includes a support portion for supporting the prefilled syringe accommodated in the packaging material and a buffer portion which is disposed further on the outside of the support portion and has an action for buffering an external force by being deformed by the external force, and a buffering material for buffering an external force by being deformed by the external force is disposed between the prefilled syringe in the packaging material and the packaging material.

2. The prefilled syringe package according to claim 1,
wherein the buffering material is one or more kinds selected from an impact absorbing gel, a polyethylene foam buffering material, soft organic polymer foam, an elastic body, and a fiber aggregate.

3. The prefilled syringe package according to claim 1 or 2,
wherein the packaging material of the prefilled syringe packages of (B) and (C) is either a packaging material having one or more of a bellows-like structure, a spring-like structure, and a sponge-like structure which are deformed by an external force as the buffer portion, or a packaging material made of a material having an action for buffering an external force by being deformed by the external force.

4. The prefilled syringe package according to any one of claims 1 to 3,
wherein the package is a form of blister packaging.

5. The prefilled syringe package according to any one of claims 1 to 4,
wherein the prefilled syringe includes a syringe main body filled with the liquid and a gasket slidable within the syringe main body, and
wherein silicone oil is applied on an inner wall surface of the syringe main body and/or a surface of the gasket.

6. The prefilled syringe package according to any one of claims 1 to 5,
wherein the liquid with which the prefilled syringe is prefilled is water for injection, a liquid containing a proteinaceous substance, a peptide, a nucleic acid, or a conjugate vaccine.

7. The prefilled syringe package according to any one of claims 1 to 6,
wherein the liquid with which the prefilled syringe is prefilled is a liquid containing biopharmaceuticals.

8. A drug kit comprising:
the prefilled syringe package according to claim 5,
a drug container in which a drug to be diluted with or dissolved or dispersed in the liquid is accommodated.
